(19)

Europäisches Patentamt
European Patent Office
Office européen des brevets

(11) **EP 3 543 911 A1**

(12) **EUROPEAN PATENT APPLICATION**

(43) Date of publication:
**25.09.2019 Bulletin 2019/39**

(51) Int Cl.:
**G06K 9/66** *(2006.01)*  **G01R 33/56** *(2006.01)*
**G06K 9/68** *(2006.01)*  **A61B 5/055** *(2006.01)*
**A61B 6/00** *(2006.01)*

(21) Application number: **18163288.6**

(22) Date of filing: **22.03.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AE Eindhoven (NL)**

(72) Inventors:
• **AMTHOR, Thomas Erik**
**5656 AE Eindhoven (NL)**

• **FUDERER, Miha**
**5656 AE Eindhoven (NL)**
• **DONEVA, Mariya Ivanova**
**5656 AE Eindhoven (NL)**
• **DE WEERDT, Elwin**
**5656 AE Eindhoven (NL)**
• **DUIJNDAM, Adrianus Joseph Willibrordus**
**5656 AE Eindhoven (NL)**
• **MEINEKE, Jan Jakob**
**5656 AE Eindhoven (NL)**

(74) Representative: **de Haan, Poul Erik et al**
**Philips International B.V.**
**Philips Intellectual Property & Standards**
**High Tech Campus 5**
**5656 AE Eindhoven (NL)**

(54) **ANOMALY DETECTION USING MAGNETIC RESONANCE FINGERPRINTING**

(57) The invention provides for a medical imaging system (100, 300, 500, 600) comprising: a memory (110) for storing machine executable instructions (120); a processor (106) for controlling the medical instrument. Execution of the machine executable instructions causes the processor to: receive (200) MRF magnetic resonance data (122) acquired according to an MRF magnetic resonance imaging protocol of a region of interest (310); reconstruct (202) an MRF vector (124) for each voxel of a set of voxels descriptive of the region of interest using the MRF magnetic resonance data according to the MRF magnetic resonance imaging protocol; calculate (204, 714) a preprocessed MRF vector (126) for each of the set of voxels by applying a predetermined preprocessing routine to the MRF vector for each voxel, wherein the predetermined preprocessing routine comprises normalizing the preprocessed MRF vector for each voxel; calculate (206, 716) an outlier map (130) for the set of voxels by assigning an outlier score (800) to the preprocessed MRF vector using a machine learning algorithm.

Fig. 1

**Description**

FIELD OF THE INVENTION

The invention relates to magnetic resonance imaging, in particular to magnetic resonance fingerprinting.

BACKGROUND OF THE INVENTION

Magnetic Resonance Fingerprinting (MRF) is a technique where a number of RF pulses, distributed in time, are applied such that they cause signals from different materials or tissues to have a unique contribution to the measured Magnetic Resonance (MR) signal. A limited dictionary of pre-calculated signal contributions from a set or fixed number of substances is compared to the measured MR signals. This can for example be used to determine intrinsic properties such as T1, T2, and B1+ values. In other examples, the comparison between a dictionary of pre-calculated signals and the measured signal can be used to determining the material composition within a single voxel. For example, if it is known that a voxel only contains water, fat, and muscle tissue the contribution from these three materials need only be considered and intra-voxel component matching can be used to accurately determine the composition of the voxel. The magnetic resonance fingerprinting technique was introduced in the journal article Ma et al., "Magnetic Resonance Fingerprinting," Nature, Vol. 495, pp. 187 to 193, doi:10.1038/nature1 1971.

SUMMARY OF THE INVENTION

The invention provides for a medical imaging system, a computer program product, and a method in the independent claims. Embodiments are given in the dependent claims.
As mentioned above, a dictionary of signals for a fixed number of substances is used in Magnetic Resonance Fingerprinting. If a particular substance is not within the dictionary, but is present within the subject being image then the matching to the dictionary could fail. This may result in errors in the calculation of the contents within a voxel. In this situation it is difficult to know if there is an abnormality within the subject, such as tumorous tissue, or if the preparation of the dictionary was insufficient.
Embodiments of the invention may provide for a means of identifying voxels which are abnormal without the prior preparation of an MRF dictionary. To do this, a machine learning algorithm is prepared. The machine learning algorithm is trained or prepared using MRF magnetic resonance data that is acquired from subjects who are not known to contain tissues that contains anomalies or abnormal anatomical structures. The machine learning algorithm is then used to calculate an outlier map for the MRF signal (also referred to as the MRF vector) for each voxel. The machine learning algorithm assigns an outlier score to each voxel. An outlier score is a numerical value which is a measure of how much the MRF vector being tested varies from the MRF vectors used to train or configure the machine learning algorithm.
The outlier map can then for example be displayed or rendered (possibly overlaid on other medical images) to help a healthcare professional identify voxels that produced abnormal or anomalous MRF vectors. This could be used to check the validity of a conventional MRF image, used for monitoring cancer growth, and/or for identifying regions of the subject to image or study further.
In one aspect the invention provides for a medical imaging system that comprises a memory for storing machine-executable instructions and a processor configured for controlling the medical instrument. The medical imaging system may take different forms in different examples. In some examples the medical imaging system is a system for processing or modifying images or data related to images. In other examples the medical imaging system may also comprise components for acquiring medical imaging data which is then processed or maybe processed into data suitable for rendering.
In the case where the medical imaging system just comprises a memory and processor and possibly other normal components of a computer or work station, controlling the medical instrument encompasses receiving processing and calculating data for generating data which is rendered or may be rendered into medical images. In other cases, the medical imaging system may comprise other components. The processor may therefore be configured for controlling these other components.
Execution of the machine-executable instructions causes the processor to receive MRF magnetic resonance data acquired according to an MRF magnetic resonance imaging protocol of a region of interest.
Execution of the machine-executable instructions further causes the processor to reconstruct an MRF vector for each voxel of a set of voxels descriptive of the region of interest using the MRF magnetic resonance data according to the MRF magnetic resonance imaging protocol. In magnetic resonance fingerprinting one or more parameters in a pulse sequence are varied and data in k-space is sampled. This sampled k-space data is then reconstructed into images. Instead of reconstructing the acquired magnetic resonance data directly into an image instead a series of images is produced. The value or values of a particular voxel for this series of images are then assembled into an MRF vector or a fingerprint for each voxel. The MRF vector may also be referred to as an MRF signal. In conventional magnetic resonance fingerprinting this MRF vector would then be compared to a dictionary comprising the MRF fingerprints or vectors for known substances or properties of the subject. In this embodiment the MRF vector which could be compared to a magnetic resonance fingerprinting dictionary is however processed differently.

Execution of the machine-executable instructions further cause the processor to calculate a pre-processed MRF vector for each of the set of voxels by applying a predetermined pre-processing routine to the MRF vector for each voxel. The predetermined pre-processing routine comprises normalizing the pre-processed MRF vector for each voxel. In this execution by the processor the MRF vector for each voxel is calculated into a pre-processed MRF vector. The predetermined pre-processing routine is a routine which is used to standardize the MRF vector for each voxel. Execution of the machine-executable instructions further cause the processor to calculate an outlier map for the set of voxels by assigning an outlier score to the pre-processed MRF vector using a machine learning algorithm.

After the MRF vector has been processed by the predetermined pre-processing routine the resulting pre-processed MRF vector is then input into a machine learning algorithm. The output of this machine learning algorithm is an outlier map. An outlier map as used herein encompasses assigning a value to each voxel which represents how close the pre-processed MRF vector is to previous measurements which are considered to be normal. The outlier map is then a measure of how well the MRF vector for each voxel matches with expected values. This may have several advantages. Firstly, the outlier map can be used to indicate which regions of a subject should be more intensely evaluated or studied by a physician or other medical professional. This embodiment also has the advantage in that it is able to identify abnormalities in a magnetic resonance fingerprinting protocol that may not be identified otherwise. In conventional magnetic resonance fingerprinting it is mentioned before that the MRF vectors are compared to a magnetic resonance fingerprinting dictionary. The construction of this dictionary requires that relevant properties of a subject such as t1, t2 or other relaxation values or the concentrations of various compounds and substances are added to the dictionary. The use of a machine learning algorithm enables the identification of abnormal portions of a subject without having to use a magnetic resonance fingerprinting dictionary.

The outlier map may for example be stored for later use or it may be rendered or displayed in different ways on displays. The outlier map may also be used in a variety of ways. The outlier map could be used for planning further imaging protocols on a subject which may for example be conventional magnetic resonance imaging or even further magnetic resonance fingerprinting studies. The outlier map maybe also useful for identifying abnormal regions of a subject for example for cancer screening or tracking the growth or remission of a cancer during therapy.

In another embodiment the medical imaging system further comprises a magnetic resonance imaging system. The memory further comprises MRF pulse sequence commands configured for controlling the magnetic resonance imaging system to acquire the MRF magnetic resonance data from the region of interest. The MRF pulse sequence commands are pulse sequence commands. The pulse sequence commands as used herein encompass commands or data which may be converted into commands that are used for controlling a magnetic resonance imaging system to acquire magnetic resonance data. In this example they are used for acquiring the MRF magnetic resonance data. The MRF magnetic resonance data is magnetic resonance data. The term MRF is used to specify particular magnetic resonance data.

The memory further comprises MRF pulse sequence commands configured for controlling the magnetic resonance imaging system to acquire the MRF magnetic resonance data from the region of interest. Execution of the machine-executable instructions further causes the processor to control the magnetic resonance imaging system to acquire the MRF magnetic resonance data. In this embodiment the medical imaging system comprises a magnetic resonance imaging system which is used to acquire the MRF magnetic resonance data.

In another embodiment execution of the machine-executable instructions further causes the processor to receive a segmentation of the voxels. The segmentation identifies a voxel type for each of the set of voxels. For example, the segmentation may identify the tissue type or organ type of particular voxels. The outlier score is at least partially assigned by using the voxel type as an input to the machine learning algorithm. In this embodiment the machine learning algorithm may be able to use the description of the voxels in the segmentation to further identify whether a particular voxel has what is considered a normal MRF vector. This may have the advantage of making the outlier map more accurate.

The segmentation of the set of voxels may take different examples in different forms. For example, the MRF magnetic resonance data maybe used with a magnetic resonance fingerprinting dictionary to generate one or more MRF magnetic resonance images. These MRF magnetic resonance images may for example be segmented or the classification of the voxels using the magnetic resonance fingerprinting dictionary may also be used as input to the machine learning algorithm. In other examples a separately acquired conventional magnetic resonance image may be segmented and this segmentation may be used.

In another embodiment the voxel type is an anatomical location derived from an annotated anatomical atlas.

In another embodiment the voxel type is a tissue type.

In another embodiment the voxel type is an organ type.

In another embodiment the voxel type may be considered to be a so called global voxel. In the global voxel case there is no differentiation used. This may be combined with other results. For example, the outlier map may be calculated using the anatomical location, the tissue type, the organ type, and/or a global voxel designation. These different combinations of outlier maps may be combined to produce a weighted or global outlier map or may be screened individually. For example, an outlier map using the global voxel definition may in some instances not identify an abnormal voxel whereas when a segmentation is used and a particular organ type is identified for each voxel it may actually show that a voxel is abnormal for that particular type of organ but is normal in the global aspect.

In another embodiment execution of the machine-executable instructions further cause the processor to reconstruct a magnetic resonance image by reconstructing the magnetic resonance image from the MRF vector for each voxel using a magnetic resonance fingerprinting dictionary. Execution of the machine-executable instructions may also include optionally calculating the segmentation of the voxels from the reconstruction of the magnetic resonance image. This embodiment may be beneficial because a magnetic resonance image produced from magnetic resonance fingerprinting is generated and also the outlier map is provided. The outlier map may for example be superimposed or compared to the magnetic resonance imaging image. This may help in the interpretation of the magnetic resonance image and/or to guide a physician or other user of the magnetic resonance image to look at particular regions identified as being abnormal by the outlier map.

In another embodiment execution of the machine-executable instructions further cause the processor to reconstruct a magnetic resonance image by reconstructing the magnetic resonance image from imaging magnetic resonance data. The imaging magnetic resonance data is descriptive of the region of interest. For example, the memory may contain imaging magnetic resonance pulse sequence commands which are used to control the magnetic resonance imaging system to acquire the imaging magnetic resonance data. This embodiment may be beneficial because the outlier map could for example be thresholded and voxels above or below a particular value may be indicated on the magnetic resonance image. This may aid in the identification of abnormal anatomical regions of the subject or regions which should be imaged or studied further.

A magnetic resonance image as used herein encompasses an image which is generated from magnetic resonance data. In the above examples the magnetic resonance image was calculated either from the MRF vectors directly or from imaging magnetic resonance data. The magnetic resonance image may map single or multiple parameters. For example the magnetic resonance image could be a T1 weighted conventional magnetic resonance image or it could also be a T1 value calculated from the magnetic resonance fingerprinting vectors. In some examples the magnetic resonance image may comprise more than one value or a multidimensional signal for each particular voxel. The magnetic resonance image could then be rendered using different colors or schemes for showing the multidimensional signal.

In another embodiment execution of the machine-executable instructions further cause the processor to identify anomalous voxels by thresholding the outlier map. Execution of the machine-executable instructions further cause the processor to render a medical image comprising the magnetic resonance image. The anomalous voxels are marked in the medical image.

In another embodiment execution of the machine-executable instructions further cause the processor to modify the machine learning algorithm by receiving training MRF magnetic resonance data acquired according to an MRF magnetic resonance imaging protocol of a training region of interest. Execution of the machine-executable instructions further cause the processor to modify the machine learning algorithm by reconstructing a training MRF vector for each voxel of the set of training voxels descriptive of the training region of interest using the training MRF magnetic resonance data according to the MRF magnetic resonance imaging protocol.

Execution of the machine-executable instructions further cause the processor to modify the machine learning algorithm by calculating a training pre-processed MRF vector for each of the training set of voxels by applying the predetermined pre-processing routine to the MRF vector for each voxel. Execution of the machine-executable instructions further cause the processor to modify the machine learning algorithm by using the training pre-processed MRF vector to adapt variable parameters of the machine learning algorithm.

In another embodiment the predetermined pre-processing routine comprises reducing the dimensionality of the MRF vector for each voxel. The MRF vector comprises a component or value for each image that is acquired in the MRF magnetic resonance imaging protocol. It may be possible to reduce the dimensionality of the MRF vector by the predetermined pre-processing routine to simplify the calculation of the outlier map by the machine learning algorithm. This may have the benefit of decreasing the calculation time of the outlier map and/or improving the reliability of the outlier map.

In another embodiment the reduction of the dimensionality of the MRF vector for each voxel comprises applying a Fourier transform to the MRF vector and truncating the Fourier transformed MRF vector above a predetermined frequency value. For example the MRF vector may have several hundred different entries. The MRF vector can be Fourier transformed using a discreet Fourier transform and then essentially applying a low pass filter to it.

In another embodiment the reduction of the dimensionality of the MRF vector comprises condensing the MRF vector using a principal components analysis algorithm. This may be useful in eliminating components of the MRF vector which do not contain relevant information.

In another embodiment the reduction of the dimensionality of the MRF vector may comprise calculating multiple relaxation times using a magnetic resonance fingerprinting dictionary. For example, a particular combination of different relaxation times chosen from T1, T2, T2* or other relaxation times may be useful in identifying abnormal voxels.

In another embodiment the predetermined pre-processing routine comprises applying a mask to remove chosen voxels from the set of voxels. For example, if voxels are within a part of the imaging volume which does not contain any tissue it may not be useful or constructive to apply or calculate the outlier map there.

In another embodiment the predetermined pre-processing routine comprises deleting chosen voxels from the set of voxels if the MRF vector is below a predetermined amplitude or a predetermined measure. For example, if a particular voxel is on the boundary of a subject and does not contain a sufficient amount of tissue or other material it may not be useful to calculate the outlier map. In some embodiments both these above methods may be applied.

In another embodiment the trained machine learning algorithm is an outlier detection algorithm. An outlier detection algorithm is an algorithm that is configured for detecting how much a measurement deviates from other measurements so as to arouse suspicion that it is generated by a different mechanism or a different physical process. This may also be referred to as anomaly detection. The outlier detection algorithm may for example be a density-based technique such as the k nearest neighbor or a local outlier factor. Neural networks or various ensemble techniques or fuzzy logic may also be used.

In another embodiment the machine learning algorithm is an isolation forest algorithm.

In another embodiment the machine learning algorithm is a k-nearest neighbors' algorithm.

In another embodiment the machine learning algorithm is a one-class support vector machine algorithm.

In another aspect the invention provides for a computer program product comprising machine-executable instructions for execution by a processor. Execution of the machine-executable instructions causes the processor to receive MRF magnetic resonance data acquired according to an MRF magnetic resonance imaging protocol of a region of interest. Execution of the machine-executable instructions further causes the processor to reconstruct an MRF vector for each voxel the set of voxels descriptive of the region of interest using the MRF magnetic resonance data according to the MRF magnetic resonance imaging protocol.

Execution of the machine-executable instructions further causes the processor to calculate a pre-processed MRF vector for each of the set of voxels by applying a predetermined pre-processing routine to the MRF vector for each voxel. The predetermined pre-processing routine comprises normalizing the pre-processed MRF vector for each voxel. Execution of the machine-executable instructions further causes the processor to calculate an outlier map for the set of voxels by assigning an outlier score to the pre-processed MRF vector using a machine learning algorithm.

In another aspect the invention provides for a method of operating a medical imaging system. The method comprises receiving MRF magnetic resonance data acquired according to an MRF magnetic resonance imaging protocol of the region of interest. The method further comprises reconstructing an MRF vector for each voxel of the set of voxels descriptive of the region of interest using the MRF magnetic resonance data according to the MRF magnetic resonance imaging protocol. The method further comprises calculating a pre-processed MRF vector for each of the set of voxels by applying a predetermined pre-processing routine to the MRF vector for each voxel. The predetermined pre-processing routine comprises normalizing the pre-processed MRF vector for each voxel. The method further comprises calculating an outlier map to the set of voxels by assigning an outlier score to the pre-processed MRF vector using a machine learning algorithm.

It is understood that one or more of the aforementioned embodiments of the invention may be combined as long as the combined embodiments are not mutually exclusive.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, microcode, etc.) or an embodiment combining software and hardware aspects that may all generally be referred to herein as a "circuit," "module" or "system." Furthermore, aspects of the present invention may take the form of a computer program product embodied in one or more computer readable medium(s) having computer executable code embodied thereon.

Any combination of one or more computer readable medium(s) may be utilized. The computer readable medium may be a computer readable signal medium or a computer readable storage medium. A 'computer-readable storage medium' as used herein encompasses any tangible storage medium which may store instructions which are executable by a processor of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the processor of the computing device. Examples of computer-readable storage media include, but are not limited to: a floppy disk, a magnetic hard disk drive, a solid state hard disk, flash memory, a USB thumb drive, Random Access Memory (RAM), Read Only Memory (ROM), an optical disk, a magneto-optical disk, and the register file of the processor. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example a data may be retrieved over a modem, over the internet, or over a local area network. Computer executable code embodied on a computer readable medium may be transmitted using any appropriate medium, including but not limited to wireless, wire line, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

'Computer memory' or 'memory' is an example of a computer-readable storage medium. Computer memory is any memory which is directly accessible to a processor. 'Computer storage' or 'storage' is a further example of a computer-readable storage medium. Computer storage is any non-volatile computer-readable storage medium. In some embodiments computer storage may also be computer memory or vice versa.

A 'processor' as used herein encompasses an electronic component which is able to execute a program or machine executable instruction or computer executable code. References to the computing device comprising "a processor" should be interpreted as possibly containing more than one processor or processing core. The processor may for instance be a multi-core processor. A processor may also refer to a collection of processors within a single computer system or distributed amongst multiple computer systems. The term computing device should also be interpreted to possibly refer to a collection or network of computing devices each comprising a processor or processors. The computer executable code may be executed by multiple processors that may be within the same computing device or which may even be distributed across multiple computing devices.

Computer executable code may comprise machine executable instructions or a program which causes a processor to perform an aspect of the present invention. Computer executable code for carrying out operations for aspects of the present invention may be written in any combination of one or more programming languages, including an object oriented programming language such as Java, Smalltalk, C++ or the like and conventional procedural programming languages, such as the "C" programming language or similar programming languages and compiled into machine executable instructions. In some instances the computer executable code may be in the form of a high level language or in a pre-compiled form and be used in conjunction with an interpreter which generates the machine executable instructions on the fly.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection maybe made to an external computer (for example, through the Internet using an Internet Service Provider).

Aspects of the present invention are described with reference to flowchart illustrations and/or block diagrams of methods, apparatus (systems) and computer program products according to embodiments of the invention. It is understood that each block or a portion of the blocks of the flowchart, illustrations, and/or block diagrams, can be implemented by computer program instructions in form of computer executable code when applicable. It is further understood that, when not mutually exclusive, combinations of blocks in different flowcharts, illustrations, and/or block diagrams may be combined. These computer program instructions may be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks.

These computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

A 'user interface' as used herein is an interface which allows a user or operator to interact with a computer or computer system. A 'user interface' may also be referred to as a 'human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, pedals, wired glove, remote control, and accelerometer are all examples of user interface components which enable the receiving of information or data from an operator.

A 'hardware interface' as used herein encompasses an interface which enables the processor of a computer system to interact with and/or control an external computing device and/or apparatus. A hardware interface may allow a processor to send control signals or instructions to an external computing device and/or apparatus. A hardware interface may also enable a processor to exchange data with an external computing device and/or apparatus. Examples of a hardware interface include, but are not limited to: a universal serial bus, IEEE 1394 port, parallel port, IEEE 1284 port, serial port, RS-232 port, IEEE-488 port, Bluetooth connection, Wireless local area network connection, TCP/IP connection, Ethernet connection, control voltage interface, MIDI interface, analog input interface, and digital input interface.

A 'display' or 'display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen,

Cathode ray tube (CRT), Storage tube, Bi-stable display, Electronic paper, Vector display, Flat panel display, Vacuum fluorescent display (VF), Light-emitting diode (LED) displays, Electroluminescent display (ELD), Plasma display panels (PDP), Liquid crystal display (LCD), Organic light-emitting diode displays (OLED), a projector, and Head-mounted display.

Magnetic Resonance (MR) data is defined herein as being the recorded measurements of radio frequency signals emitted by atomic spins using the antenna of a Magnetic resonance apparatus during a magnetic resonance imaging scan. MRF magnetic resonance data is magnetic resonance data. Magnetic resonance data is an example of medical image data. A Magnetic Resonance Imaging (MRI) image or MR image is defined herein as being the reconstructed two or three-dimensional visualization of anatomic data contained within the magnetic resonance imaging data. This visualization can be performed using a computer.

BRIEF DESCRIPTION OF THE DRAWINGS

In the following preferred embodiments of the invention will be described, by way of example only, and with reference to the drawings in which:

Fig. 1 illustrates an example of a medical imaging system;
Fig. 2 shows a flow chart which illustrates a method of operating the medical imaging system of Fig. 1;
Fig. 3 illustrates a further example of a medical imaging system;
Fig. 4 shows a flow chart which illustrates a method of operating the medical imaging system of Fig. 3;
Fig. 5 illustrates a further example of a medical imaging system;
Fig. 6 illustrates a further example of a medical imaging system;
Fig. 7 shows a flow chart which illustrates an example of a method;
Fig. 8 shows an example of an outlier map;
Fig. 9 contains a figure belonging to the SUPPLEMENT and illustrates automated wavelet balancing;
Fig. 10 contains a figure belonging to the SUPPLEMENT and illustrates the definition of HH, LH, and HL;
Fig. 11 contains a figure belonging to the SUPPLEMENT and shows a flowchart CS-SENSE including estimation of the Laplace scale quantities;
Fig. 12 contains a figure belonging to the SUPPLEMENT and shows the relationship between 1D and 2D scales; and
Fig. 13 contains a figure belonging to the SUPPLEMENT and shows estimated Laplace scales for a wavelet transforms.

DETAILED DESCRIPTION OF EMBODIMENTS

Like numbered elements in these figures are either equivalent elements or perform the same function. Elements which have been discussed previously will not necessarily be discussed in later figures if the function is equivalent.

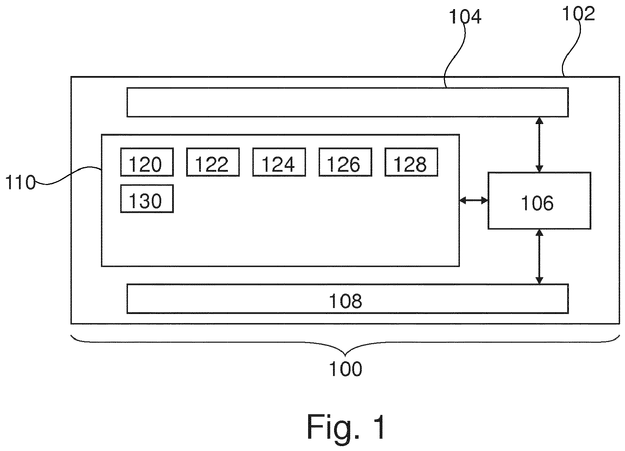

Fig. 1 illustrates an example of a medical imaging system 100. The medical imaging system 100 of Fig. 1 comprises a computer 102. The computer 102 comprises a hardware interface or network interface 104 that is shown as being connected with a processor 106. The hardware interface or network interface 104 may for example be used for exchanging data with other computer systems or other components of the medical imaging system 100. For example, if the medical imaging system 100 were to comprise a magnetic resonance imaging system the hardware interface 104 could be used to send commands to control the magnetic resonance imaging system. The processor 106 is further shown as being connected to a user interface 108 and a memory 110. The memory 110 may be any combination of memory which is accessible to the processor 106. This may include such things as main memory, cached memory, and also non-volatile memory such as flash RAM, hard drives, or other storage devices. In some examples the memory 110 may be considered to be a non-transitory computer-readable medium.

The memory 110 is shown as containing machine-executable instructions 120. The machine-executable instructions 120 are commands which enable the processor 106 to perform various functions such as the control of other components of the medical imaging system 100 and to perform various numerical and data processing tasks. The memory 110 is further shown as containing MRF magnetic resonance data 122. The MRF magnetic resonance data 122 was acquired according to a magnetic resonance fingerprinting or MRF magnetic resonance imaging protocol and is descriptive of a region of interest. The memory 110 is further shown as containing an MRF vector for multiple voxels 124 that was calculated using the MRF magnetic resonance data 122. The memory 110 is further shown as containing a pre-processed MRF vector for multiple voxels 126. The pre-processed MRF vector for multiple voxels 126 was calculated using the MRF vector 124 for multiple voxels. The memory 110 is further shown as containing a machine learning algorithm 128. The machine learning algorithm 128 is able to take the pre-processed MRF vector for the multiple voxels 126 as input and is configured to output an outlier map 130. The outlier map 130 is also shown as being stored in the memory 110.

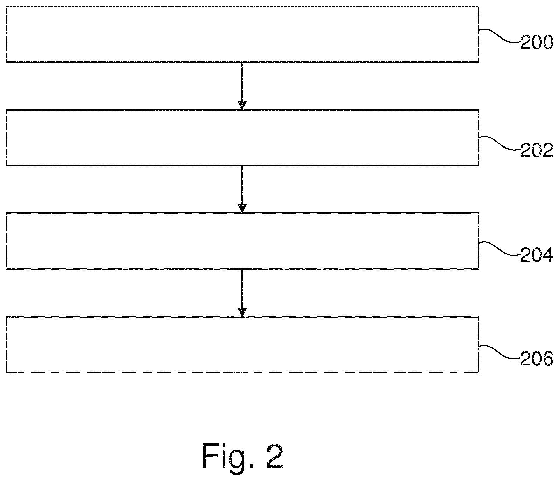

Fig. 2 shows a flowchart which illustrates a method of operating the medical imaging system 100 of Fig. 1. First in step 200 the MRF magnetic resonance data 122 is received. The MRF magnetic resonance data may for example be received via the network interface 104, via a storage medium connected to the computer 102 or it may also be acquired by a magnetic resonance imaging system controlled by the medical imaging system 100. Next in step 202 the MRF vector for multiple voxels 124 is reconstructed using the MRF magnetic resonance data 122 according to the MRF magnetic resonance imaging protocol. Next in step 204 the pre-processed MRF vector 126 for multiple voxels is calculated for each of the set of voxels by applying a predetermined pre-processing routine to the MRF vector 124 for each voxel. The predetermined pre-processing routine comprises at least normalizing the pre-processed MRF vector for each voxel. Finally, in step 206, the method comprises calculating an outlier map 130 for the set of voxels by assigning an outlier score to the pre-processed MRF vector 126 using the machine learning algorithm 128.

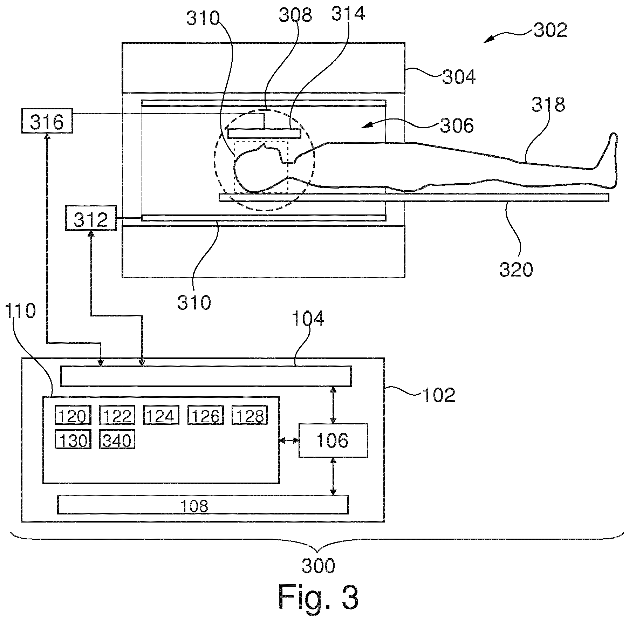

Fig. 3 illustrates a further example of a medical imaging system 300. In this example the medical imaging system further comprises a magnetic resonance imaging system 302. The magnetic resonance imaging system 302 comprises a magnet 304. The magnet 304 is a superconducting cylindrical type magnet with a bore 306 through it. The use of different types of magnets is also possible; for instance it is also possible to use both a split cylindrical magnet and a so called open magnet. A split cylindrical magnet is similar to a standard cylindrical magnet, except that the cryostat has been split into two sections to allow access to the iso-plane of the magnet, such magnets may for instance be used in conjunction with charged particle beam therapy. An open magnet has two magnet sections, one above the other with a space in-between that is large enough to receive a subject: the arrangement of the two sections area similar to that of a Helmholtz coil. Open magnets are popular, because the subject is less confined. Inside the cryostat of the cylindrical magnet there is a collection of superconducting coils. Within the bore 306 of the cylindrical magnet 304 there is an imaging zone 308 where the magnetic field is strong and uniform enough to perform magnetic resonance imaging. A region of interest 309 is shown within the imaging zone 308. A subject 318 is shown as being supported by a subject support 320 such that at least a portion of the subject 318 is within the imaging zone 308 and the region of interest 309.

Within the bore 306 of the magnet there is also a set of magnetic field gradient coils 310 which is used for acquisition of preliminary magnetic resonance data to spatially encode magnetic spins within the imaging zone 308 of the magnet 304. The magnetic field gradient coils 310 connected to a magnetic field gradient coil power supply 312. The magnetic field gradient coils 310 are intended to be representative. Typically magnetic field gradient coils 310 contain three separate sets of coils for spatially encoding in three orthogonal spatial directions. A magnetic field gradient power supply supplies current to the magnetic field gradient coils. The current supplied to the magnetic field gradient coils 310 is controlled as a function of time and may be ramped or pulsed.

Adjacent to the imaging zone 308 is a radio-frequency coil 314 for manipulating the orientations of magnetic spins within the imaging zone 308 and for receiving radio transmissions from spins also within the imaging zone 308. The radio frequency antenna may contain multiple coil elements. The radio frequency antenna may also be referred to as a channel or antenna. The radio-frequency coil 314 is connected to a radio frequency transceiver 316. The radio-frequency coil 314 and radio frequency transceiver 316 may be replaced by separate transmit and receive coils and a separate transmitter and receiver. It is understood that the radio-frequency coil 314 and the radio frequency transceiver 316 are representative. The radio-frequency coil 314 is intended to also represent a dedicated transmit antenna and a dedicated receive antenna. Likewise the transceiver 316 may also represent a separate transmitter and receivers. The radio-frequency coil 314 may also have multiple receive/transmit elements and the radio frequency transceiver 316 may have multiple receive/transmit channels. For example if a parallel imaging technique such as SENSE is performed, the radio-frequency coil 314 will have multiple coil elements.

In this example the subject, 318 is positioned such that the subject's head region is within the region of interest 309. In other examples, other parts of the subject's 318 body maybe positioned in the region of interest 309.

The transceiver 316 and the gradient controller 312 are shown as being connected to the hardware interface 104 of the computer system 102. The memory 110 is further shown as containing MRF pulse sequence commands 340. The MRF pulse sequence commands 340 enable the processor 106 to control the magnetic resonance imaging system 302 to acquire the MRF magnetic resonance data 122.

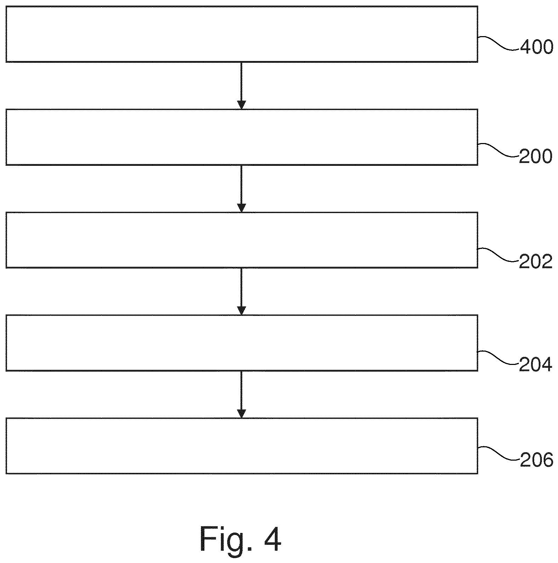

Fig. 4 shows a flowchart which illustrates a method of using the medical imaging system 300 of Fig. 3. The method starts with step 400. In step 400 the magnetic resonance imaging system 302 is controlled with the MRF pulse sequence commands 340 to acquire the MRF magnetic resonance data 122. The method then proceeds to step 200 as is illustrated in the flowchart of Fig. 2. The method of Fig. 4 then follows the method as is illustrated in Fig. 2.

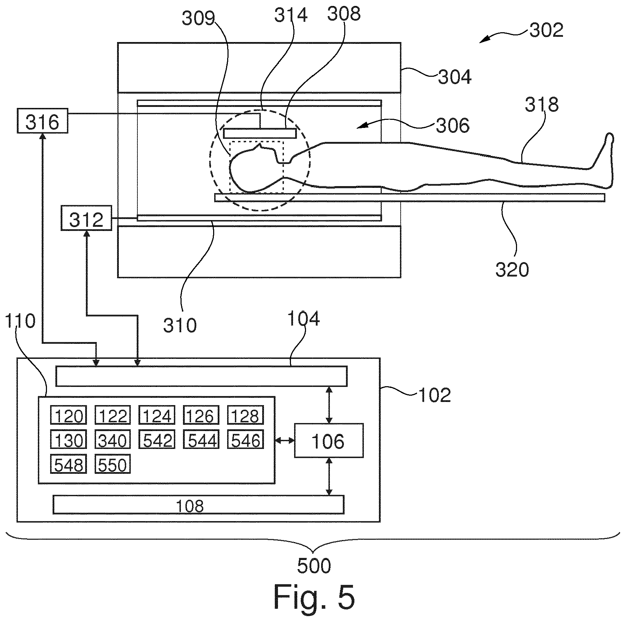

Fig. 5 shows a further example of the medical imaging system 500. The medical imaging system 500 is similar to the medical imaging system 300 in Fig. 3. The memory 110 of the medical imaging system 500 is shown as containing several additional items. The memory 110 is shown as additionally containing a magnetic resonance fingerprinting dictionary 542. The memory 110 is further shown as containing a magnetic resonance image 544 that was generated using the magnetic resonance fingerprinting dictionary 542 and the MRF magnetic resonance data 122 according to a conventional magnetic resonance fingerprinting imaging protocol.

The magnetic resonance image 544 in the memory 110 may therefore be considered a magnetic resonance fingerprinting magnetic resonance image. The memory 110 is further shown as containing an image segmentation 546 that was generated from the magnetic resonance image 544. The image segmentation 546 may for example assign a voxel type to each voxel of the MRF vector for the multiple voxels 124. The same is true for the pre-processed MRF vectors 126. The machine learning algorithm 128 may then take these voxel type identifications as a further input for determining the outlier map 130. The memory 110 is further shown as containing an identification of anomalous voxels 548 that was calculated by thresholding the outlier map 130. The memory 110 is further shown as containing a rendering of medical image 550 that is a combination of the identification of anomalous voxels 548 and the magnetic resonance image 544.

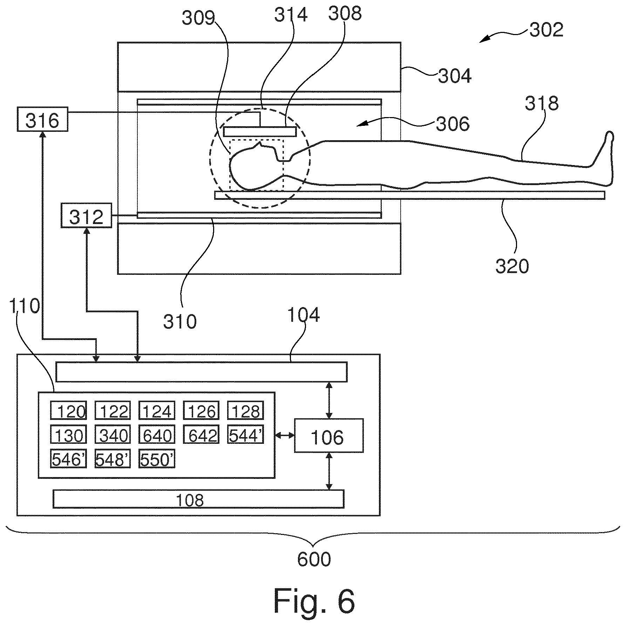

Fig. 6 illustrates a further example of a medical imaging system 600. The medical imaging system 600 in Fig. 6 is similar to the medical imaging system 300 in Fig. 3. The medical imaging system 600 has several additional items in its memory 110. The memory 110 is further shown as containing imaging pulse sequence commands 640. The imaging pulse sequence commands are instructions which enable the processor 106 to control the magnetic resonance imaging system to acquire imaging magnetic resonance data 642. The imaging pulse sequence commands 640 are according to a conventional magnetic resonance imaging protocol such as a T1, T2, or proton density weighted magnetic resonance imaging protocol. The imaging magnetic resonance data 642 may therefore be considered to be magnetic resonance data that was acquired according to a conventional magnetic resonance imaging protocol.

The memory 110 is further shown as containing a magnetic resonance image 544' that was reconstructed from the imaging magnetic resonance data 642. The memory 110 is further shown as containing an image segmentation 546' of the magnetic resonance image 544'. The memory 110 is further shown as containing an identification of anomalous voxels 548' that was made by thresholding the outlier map 130. The image segmentation 546' for example could have been used as input into the machine learning algorithm 128 to generate the outlier map 130. The memory 110 is shown as further comprising a rendering of a medical image 550' that combines the identification of anomalous voxels 548' and the magnetic resonance image 544'.

Multi-parametric MR images yield additional information about the tissue compared to a single MR contrast. MR Fingerprinting (MRF) can be considered a multi-dimensional imaging technique, since each fingerprint consists of a large number of data points. These are partly correlated, but still span a parameter space that may be larger than the two or three parameter dimensions known from standard multi-parametric imaging, depending on the number of parameters encoded in the MRF sequence.

Examples may overcome one or more of the following disadvantages:

- Screening applications usually require knowledge about a specific clinical question, so that protocols can be selected and a radiologist can be asked to search for a specific pattern. Accordingly, screening applications will always be specific and require many resources.
- Abnormalities in MRI are almost exclusively based on contrast changes in qualitative MR images and global changes in tissue properties cannot be detected.
- Multi-parametric imaging, such as Magnetic Resonance Fingerprinting (MRF), may offer some more flexibility in producing contrasts, but usually still require prior information about what contrast to produce and, in the case of MRF, what signals to expect to construct a pre-calculated dictionary.

Examples may overcome these problems by proposing a way to detect irregularities and unusual features from multi-dimensional (MRF) image data. The evaluation can be performed either in a multi-dimensional parameter space (e.g. T1, T2, diffusion) or in a multi-dimensional feature space, which can be obtained by compressing the high dimensional MRF signal without explicitly extracting the physical tissue parameters before the processing. Without the need for a model accurately including all physical effects, the method allows to find and display unusual patterns in the images. This may serve as a computer-aided diagnostic tool, which can be applied in day to day diagnostic imaging, but also would benefit screening applications as well as follow up exams so that the radiologist can be made aware of regions in the images that may need closer investigation.

Examples may comprise one or more of the following features:

- A setup for measuring MRF signals;
- An algorithm to learn features of MRF signals and to calculate anomaly scores in a multi-dimensional feature space;
- A method to calculate anomaly maps from measured MRF data;
- A method to display anomaly maps, optionally in combination with other parameter maps.

As was previously mentioned, MRF is typically performed by matching the measured fingerprint signals to a pre-calculated dictionary. This requires the model underlying the dictionary calculation to be accurate and to include all physical effects associated with the imaging system and the subject. Once the tissue parameters are estimated, the proposed analysis can be performed on this multi-parametric data. (Actually also possible if the multi-parameter mapping is not MRF.) A limitation of the above mentioned approach is that it requires the dictionary to be complete with respect to the possible substances/tissue types, because otherwise unknown tissues will be mapped to (wrong) known ones. Detection of irregularities or unusual features of the investigated tissues is only possible when all of these requirements are met. Potential errors in the estimated tissue parameters may compromise the anomaly map.

The signal anomaly evaluation (calculation of an outlier map) can also be performed on the MRF temporal signals or any other multi-dimensional feature vector directly, without explicitly applying a signal model to extract the tissue parameters. This approach is at the core of some examples:

A machine learning algorithm is trained on healthy volunteer data to be able to measure how likely it is that a probe signal is normal or has some unusual irregularity. Without any knowledge of the underlying MR sequence or the clinical question, the resulting maps show a measure of how "normal" or "unusual" the tissue is.

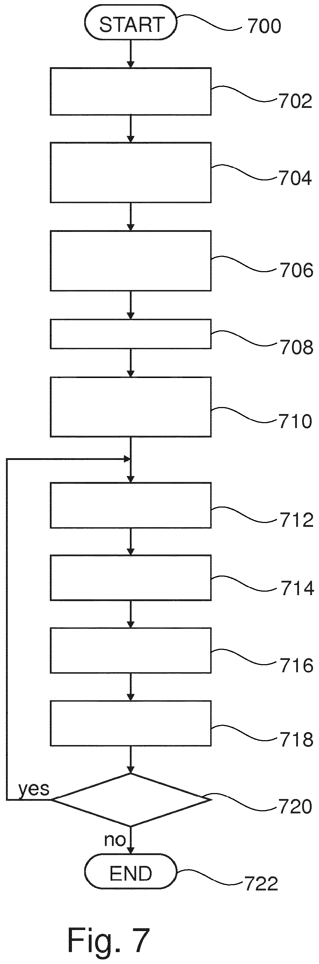

Fig. 7 shows a flowchart which illustrates an example of a method. The method starts with step 700 which is to start the method. Next in step 702 magnetic resonance fingerprinting data is acquired from healthy subjects. Step 704 is an optional step. In step 704 the data is masked according to a tissue segmentation and/or the signal strength. The data in step 704 is the MRF vector 124. Step 702 is equivalent to step 400 in Fig. 4. Step 706 is optional. In step 706 the dimensionality of the magnetic resonance fingerprint signals are reduced. Next in step 708 the MRF vector 124 is normalized. Steps 704, 706, and 708 are collectively equivalent to step 204 in Fig. 2. Next the method proceeds to step 710. In step 710 the machine learning algorithm which may also be referred to as the outlier detection algorithm is trained with the pre-processed MRF vectors 126 resulting from the application of steps 704, 706, and 708. Steps 702, 704, 706, 708 and 710 are collectively equivalent to a training or learning step for the machine learning algorithm. The steps after step 710 are equivalent to the methods illustrated in Figs. 2 and 4.

After step 710 the method proceeds to step 712. In step 712 magnetic resonance fingerprinting data is acquired from a test subject. This is equivalent to step 400 of Fig. 4. The difference between steps 712 and 702 is that in step 702 the magnetic resonance fingerprinting data was acquired from what is known as a healthy or normal subject. It is expected that the data acquired in step 702 may contain no anomalies, very few anomalies, or a reduced number of anomalies. In step 712 it is unknown if the magnetic resonance fingerprinting data was acquired from a subject which has any anomalies in its anatomy. After step 712 the method proceeds to step 714. Step 714 is equivalent to step 204 in Figs. 2 and 4. The steps performed in step 714 are equivalent and equal to the steps 704, 706, 708 that were performed earlier. Next in 716 an anomaly score is calculated for each voxel 716. Step 716 is equivalent to step 206. The anomaly score is equivalent to the outlier score. The method then proceeds to step 718. In step 718 the anomaly map, which may also be referred to as the outlier map, is visualized. Step 718 is optional. The outlier map or anomaly map may also be stored in a memory for later use. The method then proceeds to step 720. Step 720 is a decision box. The question is are there more subjects to process. If the answer is "no" then the method proceeds to step 722 and the method of Fig. 7 ends. If the answer is "yes" the method proceeds back to step 712 and steps 712, 714, 716, and 718 are performed again.

Preparation of training magnetic resonance data:

First, MRF data / multi-parametric MR data is acquired from several healthy subjects. Voxels to be included in the training data set are selected by signal amplitude (low-amplitude signals, e.g. outside the subject, are rejected) and optionally by tissue type (this can be achieved by calculating standard MRF classification maps, calculating standard MRF parameter maps and selecting a parameter range, manual segmentation, or automated model-based segmentation).

The signals selected as a training data set can optionally be compressed through dimensionality reduction. For example, in the case of spiral sampling (with periodically rotated spirals), a Fourier transform of the signals with subsequent selection of the low-frequency components can be used to eliminate sampling artefacts while maintaining most of the encoded tissue information.

The reduced signal vectors are still multi-dimensional and may span ten to several hundred dimensions.

The signals are then normalized to allow distance measures independent of the abundance of the substance in the voxel. Optionally, the vector elements may be transformed to absolute numbers to match the requirements of some machine-learning algorithms. Alternatively, the training data can be the estimated tissue parameters, T1, T2, proton density, diffusion, and/or other parameters.

Training of the machine learning algorithm:

Once the data set is prepared, a machine learning algorithm is trained or modified. Some example algorithms that can be used are:

- The Isolation Forest algorithm:

This algorithm is well-suited for multi-dimensional cluster and outlier analysis. Once trained, it calculates a score for each test vector that specifies the conformity with the training data.

- The k-NN (k nearest neighbors) algorithm:

For each test vector, this algorithm returns the distances to the k nearest neighbors from the training data set. In the context of some examples, the mean distance of the k nearest neighbors (with k=3 for example) would serve as a measure for the anomaly of the test vector.

- The one-class support vector machine algorithm:

This algorithm uses a set of training examples to define a boundary between inliers and outliers. The test data points are then classified as belonging to one of two categories. This algorithm is an example of a non-probabilistic binary classifier that uses a model that assigns new examples to one category or the other.

Analysis of test data sets:

The signals measured from a test subject (patient to be screened) are prepared in the same way as the training signals (Voxel selection, dimensionality reduction, normalization).

The signals are then tested using the trained machine learning algorithm, which yields an anomaly score for each voxel.

Furthermore, the location of a voxel, e.g. frontal lobe, hippocampus, etc., could be taken into account for assessing the anomaly-score of a given signal. This can be achieved by warping the acquired data to an annotated atlas, e.g. using mesh-based image warping in combination with model-based segmentation.

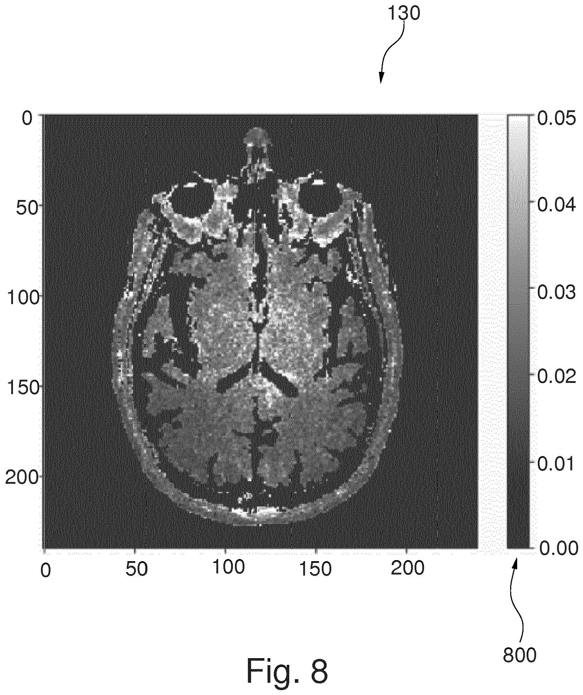

Fig. 8 illustrates an example of an outlier map 130 which may also be referred to as an anomaly map. The Fig. 130 is a grayscale image which assigns an outlier score 800 to each voxel in the slice shown. The outlier score 800 is shown by the grayscale bar to the right of the image 130. The outlier score 800 may also be referred to by the term as anomaly score. The outlier or anomaly map of Fig. 8 was calculated using a 3-NN algorithm: Voxels containing liquids or very low signal have been excluded. The values indicate the mean distance of the MRF signal from the three closest nearest neighbors selected from the training data sets. In this case, this is the Euclidian distance of 30-dimensional normalized feature vectors. Since this is a healthy test subject, no significant anomalies are visible. This visualization could be overlaid, possibly in color, on standard contrast or parameter maps.

In another example, the anomaly maps are analyzed statistically or via image analysis to yield a proposal for the radiologist. This proposal could then be displayed as a marked image or a text, e.g. "High probability for irregularity in the frontal lobe region" or "Please check existence of lesions in region _.".

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measured cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

LIST OF REFERENCE NUMERALS

| 100 | medical imaging system |
| 102 | computer |
| 104 | hardware or network interface |
| 106 | processor |
| 108 | user interface |
| 110 | memory |
| 120 | machine executable instructions |
| 122 | MRF magnetic resonance data |
| 124 | MRF vector for multiple voxels |
| 126 | preprocessed MRF vector for multiple voxels |
| 128 | machine learning algorithm |
| 130 | outlier map |
| 200 | receive MRF magnetic resonance data acquired according to an MRF magnetic resonance imaging protocol of a region of interest |
| 202 | reconstruct an MRF vector for each voxel of a set of voxels descriptive of the region of interest using the MRF magnetic resonance data according to the MRF magnetic resonance imaging protocol |
| 204 | calculate a preprocessed MRF vector for each of the set of voxels by applying a predetermined preprocessing routine to the MRF vector for each voxel |
| 206 | calculate an outlier map for the set of voxels by assigning an outlier score to the preprocessed MRF vector using a machine learning algorithm |
| 300 | medical imaging system |
| 302 | magnetic resonance imaging system |
| 304 | magnet |
| 306 | bore of magnet |
| 308 | imaging zone |
| 309 | region of interest |
| 310 | magnetic field gradient coils |
| 312 | magnetic field gradient coil power supply |
| 314 | radio-frequency coil |
| 316 | transceiver |
| 318 | subject |
| 320 | subject support |
| 340 | MRF pulse sequence commands |
| 400 | control the magnetic resonance imaging system to acquire the MRF magnetic resonance data |
| 500 | medical imaging system |
| 542 | magnetic resonance fingerprinting dictionary |
| 544 | magnetic resonance image |
| 544' | magnetic resonance image |
| 546 | image segmentation |
| 546' | image segmentation |
| 548 | identification of anomalous voxels |
| 548' | identification of anomalous voxels |
| 550 | rendering of medical image |
| 550' | rendering of medical image |
| 600 | medical imaging system |
| 640 | imaging pulse sequence commands |
| 642 | imaging magnetic resonance data |
| 700 | Start |
| 702 | Acquire MRF data from healthy subjects |
| 704 | A) Mask data according to tissue segmentation |
| 706 | B) Optionally reduce dimensionality of MRF signals |

708    C) Normalize signals
710    Train Outlier detection algorithm with selected / reduced MRF signals
712    Aquire MRF data from test subject
714    Prepare data according to steps A, B, C
716    Determine anomaly score for each voxel
718    Visualize anomaly map
720    More Subjects?
722    End
800    outlier score

SUPPLEMENT

Supplement Title: Automated Wavelet Balancing

Supplement Authorship: Miha Fuderer, Adri Duijndam, and Elwin de Weerdt

The authorship of this Supplement differs from rest of this document. The authors of the SUPPLEMENT are not authors of the rest of this document. Text between the header SUPPLEMENT above and the CLAIMS below belongs to the SUPPLEMENT.

**Abstract:**

Compressed Sensing MRI is a valuable technique to speed up acquisition time; however, it requires a factor to balance the influence of the wavelet-constraint relative to the data-fidelity constraint. This factor is often 'manually' set. Examples suggests to automatically estimate that factor. The optimal balancing factor is calculated based on the statistics of a set of individual wavelet-scales.

**Detailed description**

Background of examples

Compressed Sensing (CS) is a technique that has promising applications in MRI. It allows for sampling a reduced set of data points in k-space while still reconstructing a good resulting image. This possibility is based on the knowledge that the resulting image is compressible. A CS algorithm applies a nonlinear operation in a domain where the image representation is assumed to be sparse. Typically, the domain is the Wavelet-transform of the image, and the nonlinear operation consists of 'threshholding', which consists of putting those wavelet-elements to zero that are below a certain threshold (and by that, in practice, removing more noise and artefacts than relevant signal).

Typically, the mathematical problem is expressed as the minimization of the following function:

$$F(\mathbf{p}) = \frac{1}{2}\|\mathbf{m} - \mathbf{A}\mathbf{p}\|_2^2 + \lambda\|\mathbf{W}\mathbf{p}\|_1. \qquad (0.1)$$

Here, p is the image-vector to be reconstructed; m is the measured data, A =MFS is an encoding matrix containing , with S containing coil sensitivities, F represents a Fourier transformation and M represents a matrix describing (irregular) subsampling. Actually the matrix A contains regularization information as well, see a more detailed description below.

**W** represents the Wavelet transform and $\|.\|_1$ should be read as "the L1-norm" (sum of absolute values). The factor "$\lambda$" will be discussed in the sequel. Note that regularization is left out of this formulation but can easily put in, without loss of any essential element, see the appendix, where it is taken along.

In essence, the first term tells that we want to find a **p** that fits the data; the second term tells that **p** should be compressible. And $\lambda$ weighs one against the other. Needless to say that the resulting image will strongly depend on the choice of $\lambda$. In current practice, the factor $\lambda$ is set ad-hoc, i.e. manually tuned for a specific application.

Problems or disadvantages overcome by some embodiments

Our aim is to derive, from the image or data properties, a favourable choice of $\lambda$, and to do so in a large range of circumstances (compression factors, resolutions, contrasts, signal-to-noise conditions etcetera), without requiring the user to set this value.

The main element(s) of some examples

Examples may contains the following elements, which may be applied independently from each other:

- The factor $\lambda$ is set depending on the histogram statistics of the wavelet components. More specifically, the histogram statistics of the finest wavelet component is derived from the statistics of the coarser scales.
- To preserve resolution, we limit the factor $\lambda$ to a value that ensures that the signal-energy in the finest wavelet scales is not reduced further than a fixed fraction (e.g., to 90%) of the signal-energy that we would obtain without the application of the sparsifying term.

Detailed description of how to build and use some examples

A particular example

The following processing steps are applied for each slice and for each iteration of a Nesterov/Fista optimization scheme. See the Appendix for a detailed description on the calculations required within the wavelet thresholding step:

- Calculate the Laplace scales of the image, for each wavelet scale, by an analysis of the histogram of the values of each wavelet scale. The Laplace scale is called "b" here. A correction factor for the amount of background is taken into account in this step.
- Estimate the parameters describing the exponential relationship between Laplace scales and wavelet scales. A combined estimation procedure is used here, where the so-called HH, LH and HL wavelet domain parts are separated in the exponential model, but estimated in an integrated fashion. Anisotropy properties of the voxel sizes are taken into account.
- Derive the lowest meaningful Laplace scale from this exponential relationship. See the appendix for details. These three steps together lead to a refined (and stable) estimate of b pertaining to the lowest wavelet scale.
- Derive the wavelet threshold by $t = \frac{\sigma^2}{bc}$, where $\sigma$ is the standard deviation of the noise in the input data, and c is the maximum eigenvalue of the matrix $\mathbf{A}^H \mathbf{A}$ (which is known).
- Calculate the upper allowable limit to the threshold as $\frac{3bf}{2cR}$, here, $f$ is a system-tunable factor, e.g. 0.1. $R$ is the speedup factor (the ratio of sample density relative to the nominal required sample density).
- Set the threshold to the maximum of the two aforementioned values, i.e. $t = \min(\frac{\sigma^2}{bc}, \frac{3bf}{2cR})$.
- Apply the wavelet thresholding using this threshold t.

Possible alternatives or refinements

- The factor $\lambda$ is set differently per wavelet-scale.
- The thresholding function within the wavelet-step is a function in-between hard-thresholding and soft-thresholding.

More specifically, we calculate a 'subtraction value, $t_s = t \cdot p \cdot |\hat{x}'|^{P-1} \frac{\hat{x}'}{|\hat{x}|}$. (Here, $\overset{\wedge}{x}$ the wavelet-component before the thresholding). The value of $p$ is preferably chosen as 0.7; the essence of this idea is that it is chosen as nonzero, but smaller than 1. The result of the thresholding is calculated as $\hat{\hat{x}}' = \begin{cases} \hat{x}' - t_s & |\hat{x}'| > t_s \\ 0 & |\hat{x}'| \leq t_s \end{cases}$.

Detectability

Detectability of the details is an issue. However, it can be appreciated from the user-interface that the system does not require manual setting of the wavelet parameter.

Applications of some examples

MRI

Appendix of the SUPPLEMENT - background of "detailed description" for the Supplement Formulation of the inverse problem

Let the forward model of the measured data be given by,

$$m = MFSp + n \qquad (0.2)$$

with the following definitions:

- **m** is the vector of measured data, augmented with zero values at the k-space positions that are not measured.
- **p** is the vector containing the pixels of the image,
- **S** is the matrix containing coil sensitivities,
- **F** is the matrix representing the (full) Fourier transformation.
- **M** is a block diagonal matrix containing values of one on the diagonal for measured k-space positions and values of zero otherwise (these weights can be generalized).
- **n** is a vector with noise

The matrices **S, F** and **M** can be combined in the encoding matrix E= **MFS.** The noise is assumed to Gaussian distributed with zero mean. In the description we will take the noise to be fully normalized so the noise covariance matrix equals the identity matrix.
The "model" of the familiar principle of regularization has a similar but simpler mathematical structure:

$$0 = Ip + n_r \qquad (0.3)$$

specifying that the expected value of the (complex valued) image pixels is zero. The uncertainty is taken to be Gaussian and its properties are specified in the diagonal covariance matrix **R**. This matrix will have low values on the diagonal for voxels in the background, which effectively stabilizes the inverse problem and leads to higher SNR.
The wavelet transform of the image **p** is given by the operation **Wp.** The prior information with respect to sparsity of the wavelet transform is formulated through the following probability density function

$$p(\mathbf{p}) \propto \exp(-\|\mathbf{B}^{-1}\mathbf{W}\mathbf{p}\|_1) \qquad (0.4)$$

The uncertainties here are taken to have a Laplace distribution, representing the assumption that the wavelet coefficients are sparsely distributed. The uncertainties are given through the diagonal matrix **B.**
Given these ingredients and using a Bayesian formalism, the reconstruction problem boils down to finding the minimum of the following function:

$$F(\mathbf{p}) = \frac{1}{2}\|\mathbf{m} - \mathbf{E}\mathbf{p}\|_2^2 + \frac{1}{2}\|\mathbf{R}^{-1/2}\mathbf{p}\|_2^2 + \|\mathbf{B}^{-1}\mathbf{W}\mathbf{p}\|_1. \qquad (0.5)$$

Though the Laplace scales are different per wavelet scale, in the optimization procedure (actually, in the included soft thresholding step) a constant value is used, using the form **B** = $\lambda^{-1}$**I**.
Solving this optimization problem requires a dedicated algorithm, which is described in the sequel.

Optimization algorithm

The optimization problem formulated above can be written in a more compact notation as:

$$F(\mathbf{p}) = \frac{1}{2}\|\mathbf{m} - \mathbf{Ap}\|_2^2 + \lambda\|\mathbf{Wp}\|_1 \,, \qquad (0.6)$$

where the data vector is redefined as:

$$\mathbf{m} \leftarrow \begin{bmatrix} \mathbf{m} \\ \mathbf{0} \end{bmatrix}, \qquad (0.7)$$

and, correspondingly,

$$\mathbf{A} = \begin{bmatrix} \mathbf{E} \\ \mathbf{R}^{-\frac{1}{2}} \end{bmatrix}. \qquad (0.8)$$

The optimization problem ((0.6) can be solved with an iterative soft-thresholding algorithm, see the description of FISTA (Fast Iterative Soft Thresholding Algorithm)
For i = 1,2,...,imax do:

$$\mathbf{z}_i = \mathbf{p}_i + \frac{i-1}{i+2}(\mathbf{p}_i - \mathbf{p}_{i-1}) \qquad (0.9)$$

$$\mathbf{p}_{i+1} = \mathbf{W}^{-1} S_{\lambda/c} \mathbf{W} \left( \frac{1}{c} \mathbf{A}^H (\mathbf{m} - \mathbf{A}\mathbf{z}_i) + \mathbf{z}_i \right)$$

where S is the element-wise soft-thresholding operator

$$S_\mu(x) = \begin{cases} 0 & |x| \le \mu \\ \frac{|x|-\mu}{|x|} & |x| > \mu \end{cases}, \qquad (0.10)$$

and c should be smaller than or equal to the maximum eigenvalue of the matrix $\mathbf{A}^H\mathbf{A}$.

Wavelet thresholding

First some basic properties of the wavelet transform of real world images are introduced. Then the estimation of some of the related properties is discussed. The treatment of the application to the wavelet thresholding follows.
An explanation of the wavelet transform is beyond the scope of this document. The reader is referred to https://en.wikipedia.org/wiki/Wavelet_transform and
https://en.wikipedia.org/wiki/Discrete_wavelet_transform, and references there.

Choice of the wavelet transform

In principle examples may be applicable to any type of wavelet transform used.The Daubechies family of wavelets and particularly the D4 wavelet are well known and popular choices. See also https://en.wikipedia.org/wiki/Discrete_wavelet_transform.
One characteristic of this form of wavelet transform is that it is not shift invariant and is directionally selective. Adverse effects related to (the absence of) these properties have been seen on practical data sets. One option to overcome these limitations is the use of the dual tree complex wavelet transform.

Wavelet transform properties

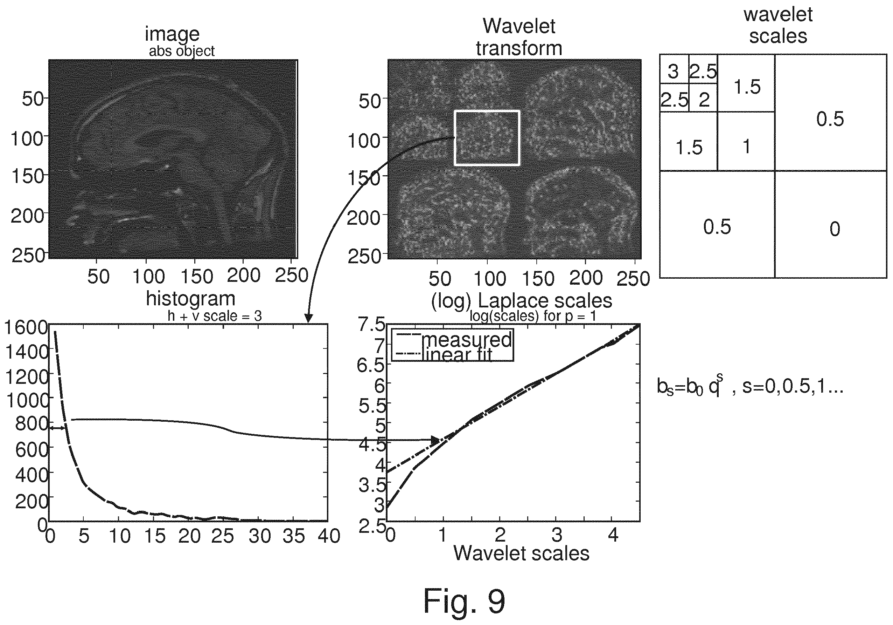

In order to introduce this topic, let us consider an image and its corresponding wavelet transform, shown in Fig. 9 (top left and middle). Two things can be observed: (1) for each wavelet scale, many coefficients have low absolute values and (2) the amplitude of the coefficients increases for coarser scales.

Fig. 9 shows An image (top left) and the absolute values of the wavelet transform (top right). The definition of the wavelet scales is shown top right. A histogram of the set of coefficients of one of the wavelet scales is shown in the bottom left. When the width of the distribution is plotted as function of the wavelet scale, the bottom right picture results. Note the logarithmic vertical scale.

Let us look at these two observations in more detail in the next sections.

Laplace distribution of wavelet coefficients

With respect to the first observation above, if we generate a histogram from the coefficients (see the lower left picture of Fig. 9) for one particular wavelet scale, we see that it roughly follows a Laplace distribution. For a single variable x this distribution is given by

$$p(x|\mu, b) = \frac{1}{2b} e^{-\frac{|x-\mu|}{b}} \qquad (0.11)$$

with $\mu$ a location parameter and b the scale parameter. (In order to avoid confusion with the "wavelet scale", this scale parameter will be mostly referred to as "Laplace scale".) With this distribution we can model the first observation above: for each wavelet scale, many coefficients have relatively low values. Significant coefficients are "sparse". This observation is used in the sparsity constraint as formulated in the minimization problem, and is one of the most essential elements in the CS-SENSE algorithm. It is also for this reason that the wavelet transform is chosen, apart from computational considerations. This image is sparse in the wavelet domain, and this information is used to constrain the output space of the SENSE solution. In principle of course, any transformation that has a sparse representation of the image could be used.

Now from the inverse problem formulation it is clear that we need to know the parameters of the Laplace distribution (actually $\mu$ can be neglected, so is mainly pertains to the Laplace scale $b$), for each wavelet scale in order to be able to use this sparsity information. The estimation of these parameters is discussed below. It turns out to be very helpful to use a relationship between the wavelet scales for this purpose.

Multiscale relationship

Let us define the wavelet scale *s* as in Fig. 9 (top right). It is defined as being zero at the finest scale, increasing by 1 for each coarser scale. The intermediate scales 0.5,1.5 etc. are the horizontal or vertical components of the wavelet transform. It can be seen that the Laplace scales are larger for coarser wavelet scales. The dependency on the wavelet scales can be formulated through:

$$b_s = b_0 q^s, \qquad s = 0,0.5,1,... \qquad (0.12)$$

This relationship (with q ≈ 3) has been found to describe the data remarkably well, except for the finest scales, see also the example of Fig. 9 for an illustration of this.

Since this piece of prior knowledge is essential in the approach to wavelet thresholding, the theoretical background of it will be discussed in a bit more detail in the next section. Relation between wavelet domain and k-space and background of the multi-scale relationship In order to provide a rational for (0.12), it is important to understand the relationship between the wavelet scales and position in k-space. A good way to show this relation is to study the Fourier transform of an image that is built up of just one wavelet coefficient.

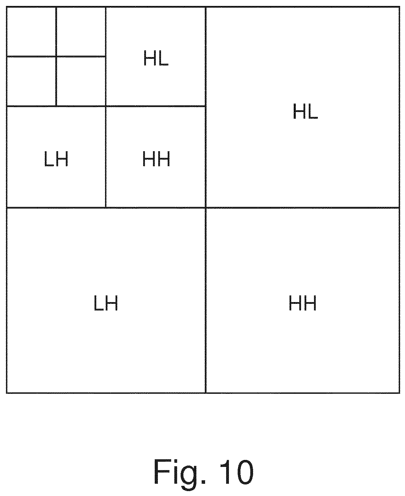

First we need to realize that the wavelet coefficients are located in the different parts, HH, LH and HL of the wavelet domain, see Fig. 10 below. In the indication of these parts, the first character corresponds with the first axis and the second character with the second axis. Consider the first axis as horizontal and the second axis as vertical. The "L" stands for low pass filtered and the "H" for high pass filtered. The different parts, which are of course repeated for the different wavelet scales, correspond to different "wavelet types". In the sequel of this document we will often refer to these distinct parts as "types", of which we have the three distinct flavors as mentioned above.

Fig. 10 shows the Definition of the HH, LH and HL parts of the wavelet domain (repeated for each wavelet scale).

Now consider a disk in image space. The Fourier transform of such a disk with radius r is given by

$$P(k) = \frac{\sqrt{3r}}{4k} J_1(2\pi kr)$$

$$P(k) \propto \underset{\text{overall decay}}{k^{-1.5}} \cos\left(k - \frac{3\pi}{4}\right) \qquad k \gg \frac{3}{8\pi r} \qquad\qquad (0.13)$$

So, decreasing the wavelet scale with one, corresponds to increasing the position in k-space with a factor of two, which in turn corresponds to a reduction in amplitude of a factor of $2^{1.5} \approx 2.83$. Deviations of this factor in practice stem from different anatomy shapes and finite slice thickness. The effect of zero padding in k-space, as well as the effect of anisotropic resolution, is discussed in the next section.

Estimation of the Laplace scales

The Laplace scales are not known prior to the reconstruction of a CS-SENSE scan because they are object dependent. There is no pre-scan or general information available to derive these values upfront hence these properties have to be estimated as an integral part of the CS-SENSE algorithm. And they have to be estimated from (partially) reconstructed images. There are two issues that hamper the estimation of these properties.

- In the very beginning of the iterative scheme, or during early iterations, we only have an incomplete reconstruction. The images will still contain aliasing artefacts, apart from noise.
- Even at later, and the final, iterations, the image will still be contaminated with noise and remaining artifacts (possibly incomplete unfolding, but also artifacts due to imperfect coil sensitivities, motion and other common causes of artifacts. These will particularly influence the finer wavelet scales, so those cannot be estimated reliably.

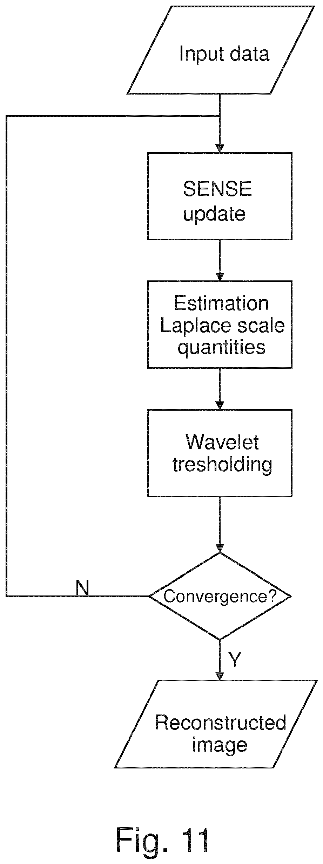

The following scheme, shown in Fig. 11, should overcome these issues. The wavelet properties are estimated during the iterations and for each iteration. The estimates will be inaccurate for early iterations and become more accurate as the iterations proceed. The Laplace scales are estimated for all wavelet scales. Subsequently, the multi-scale relationship will be used in order to accurately estimate the Laplace scale for the finest wavelets scale(s) from the coarser wavelet scales. This last aspect reduces the influence from random noise and the artifacts mentioned above.

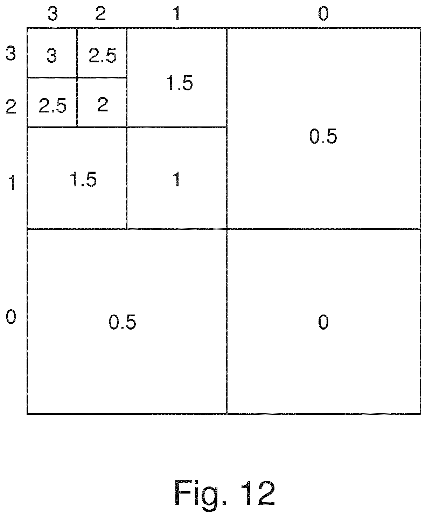

Fig. 12 shows a flowchart CS-SENSE including estimation of the Laplace scale quantities The details of these steps are discussed below.

Estimation of the individual Laplace scales

Basic estimation

Given the one-dimensional Laplace distribution, and a set of $N$ independent and identically distributed samples $x_1 ... x_N$, the maximum likelihood estimator $\hat{\mu}$ is the sample median, and the maximum likelihood estimator of $b$ is

$$\hat{b} = \frac{1}{N} \sum_{i=1}^{N} |x_i - \hat{\mu}| \qquad\qquad (0.14)$$

For the complex valued images as occurring in MRI, it is assumed that the sample median is sufficiently close to zero, so that it can be neglected. The estimator for the Laplace scale then simply is $\hat{b} = \frac{1}{N} \sum_{i=1}^{N} |x_i|$. An alternative approach of using the 90% percentile of the sorted samples for the estimation of b has also been proposed, but we have found that the maximum likelihood estimator is more accurate. Moreover, it does not require a sorting of the absolute values of the samples and is therefore more efficient.

Correction for background value

It is desirable to have the estimates of the Laplace scales to be independent of the amount of zero padding that is done in image space. After all, the properties of the image contents are not changed just by adding edges with zeros values around it. Such a zeropad action occurs in order to extend the image to a matrix size that is suitable for the wavelet transform.

Now, if the fraction of zero valued samples is denoted by the symbol $f$ than the mean of the wavelet coefficients is reduced by a factor $(1 - f)$ compared to the situation without zero padding. Correspondingly, in order to arrive at "zero-padding independent" Laplace scales, the mean values from the previous subsection need to be divided by $(1 - f)$.

A similar reasoning holds for all samples that are in the background (not part of the object). So, more in general, the raw estimated Laplace scales need to be divided by $(1 - f)$, with f the ratio of samples in the background and the total number of samples.

Estimation of relation between levels

For the CS-SENSE scheme, q and $b_0$ are estimated by first estimating the Laplace scale using equation (0.14) for all wavelet scales (and the three parts HH, LH and HL), including background mask correction. From these Laplace scales and relation ((0.12), q and $b_0$ are estimated. There are several aspects that need to be taken into account in order to arrive at a "picture" of the wavelet scale dependent Laplace scales that depends only on intrinsic object properties and is independent of acquisition or processing dependent details. These latter aspects cover:

- Zero padding in k-space (interpolation)

- Anisotropic resolution

Zero padding in k-space

Consider, as an example, zero padding with a factor of two along a particular direction. Since this corresponds with interpolation with a factor of two, it is clear that the contents of the image in the wavelet domain shift one scale up, compared to the situation without interpolation. See section 0. This needs to be incorporated in the definition of the wavelet scale.

Let us add a bit more detail to the specification of the wavelet scales, and particularly the relation between one-dimensional and two-dimension wavelet scales.

Fig. 12 shows a relation between 1D and 2D wavelet scales. The ID scales are depicted along the two axes.

Let us define the wavelet scales, for a ID wavelet transform, for either x or y scales as

$$s_{x,y} = 0,1,2, \ldots \qquad (0.15)$$

We can now define the wavelet scales for a 2D transform as

$$s_{xy} = \frac{s_x + s_y}{2}$$

$$(0.16)$$

$$s_{x,y} = 0.5, 1, 1.5, \ldots$$

For increasing zeropadding in k-space (or interpolation in image space), it is clear that the wavelet scales that come out of the wavelet transform correspond to decreasing "physical size". The "true finest scale" of the wavelet transform as used in determination of the energy preserving threshold should correspond to the acquisition voxel size. Since the wavelet scales have a logarithmic relation to the matrix size (and thereby the voxel size) given a fixed field of view, the "true scales" along one direction (taking x as an example) can be defined as:

$$s_{x,true} = s_x - \log_2 f_{x,zp}$$

$$s_x = 0,1,2, \ldots$$

$$(0.17)$$

$$f_{x,zp} = \text{zero pad factor in k-space, along x} = \frac{\Delta x_{acq}}{\Delta x_{rec}} = \frac{N_{x,rec}}{N_{x,acq}}$$

With the definition of the 2D scales (0.16) we get

$$
\begin{aligned}
s_{true} &= \frac{s_{x,true} + s_{y,true}}{2} \\
&= \frac{s_x + s_y}{2} - \frac{\log 2(f_{zp,x}) + \log 2(f_{zp,y})}{2} \\
&= s - \log 2(\sqrt{f_{zp,x} f_{zp,y}})
\end{aligned}
\qquad (0.18)
$$

$$
s = 0, 0.5, 1, L
$$
.

This implies, for example, that with a zero pad factors of 2 for both dimensions, the "true scales" are reduced by one compared to the "numerical scales". This ensures a consistent estimation of the Laplace scales, more or less independent of the amount of zero padding.

Anisotropic resolution and multiscale estimation

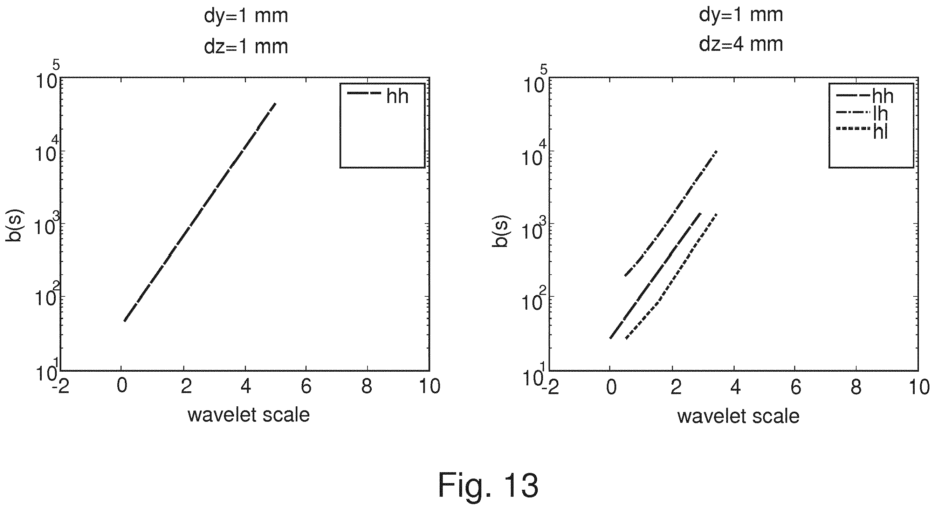

Let us now turn to the topic of anisotropic resolution and the consequences on the wavelet transform. And more particularly on which wavelet scale (which "box" on the wavelet domain) we would like to base the threshold on. Consider a synthetic example of anisotropic resolution in comparison with isotropic resolution for a disk. Fig. 13 shows the corresponding estimated Laplace scales. It is clear that due to the anisotropic resolution, the Laplace scales are significantly different for the three different types (HH, LH and HL) of scales.

Fig. 13 shows Estimated Laplace scales for the wavelet transforms. For the HH, LH and HL boxes (see Fig. 12 for the definitions).

In order to handle this correctly, the multi-scale model (0.12) is used separately for each "wavelet type". The base of the exponential function q is assumed to be identical for the types. So we use the model

$$
\begin{aligned}
b_s^{HH} &= b_{HH} q^{s_{HH}}, \quad s_{HH} = 0, 1L \\
b_s^{LH} &= b_{LH} q^{s_{LH}}, \quad s_{LH} = 0.5, 1.5L \\
b_s^{HL} &= b_{HL} q^{s_{HL}}, \quad s_{HL} = 0.5, 1.5L
\end{aligned}
\qquad (0.19)
$$

where for the *s* values, the values after correcting for zero padding are used. The unknown parameters can estimated by taking the logarithm on both sides of these equations:

$$
\begin{bmatrix}
\ln b_s^{HH} \\
M \\
M \\
\ln b_s^{LH} \\
M \\
M \\
\ln b_s^{HL} \\
M \\
M
\end{bmatrix}
=
\begin{bmatrix}
1 & & s_{HH} \\
M & & M \\
1 & & M \\
& 1 & s_{LH} \\
& M & M \\
& 1 & M \\
& & 1 & s_{HL} \\
& & M & M \\
& & 1 & M
\end{bmatrix}
\begin{bmatrix}
a_{HH} \\
a_{LH} \\
a_{HL} \\
q\%
\end{bmatrix}
\qquad (0.20)
$$

with

$$a_{HH} = \ln b_{HH}$$
$$a_{LH} = \ln b_{LH}$$
$$a_{HL} = \ln b_{HL} \qquad (0.21)$$
$$q\!\!\!/ = \ln q \qquad ,$$

and solving (0.20) in a weighted least squares sense.

Estimation details

A couple of practical aspects of the estimation procedure need to be covered:

- Number of wavelet levels
- Weighting of levels
- Alternative to least squares

All wavelet levels are used in the estimation process. But weights are applied defined as

$$w(0) = 0$$
$$w(1) = 0.5$$
$$w(2) = 1 \qquad (0.22)$$
$$w(s) = 2^{-\frac{s-2}{2}} \qquad s > 2$$

So effectively the finest scale ($s = 0$) is not used.

L1 fitting could be used as an alternative to least squares fitting.

Selection of the threshold

Two aspects need to be considered when determining the precise form of the sparsity term in the inverse problem to be solved. The first one is whether the threshold should vary per wavelet type and scale or whether a constant level is required. The second one is the item of "wavelet balancing", the level of thresholding (relative to the data fit and regularization terms). These topics are described in the next sections.

Single or multi scale thresholding

Given the material above and the general formulation of the inverse problem, one would expect that the best reconstruction would be one that would allow a threshold 1 $\alpha$ 1/b that would decrease for increasing wavelet scale (which show increasing b). While this may be true from a pure quantitative viewpoint, the effect of level dependent thresholding is that the noise becomes colored, which makes the images look artificial. They are not pleasing to the eye. In this case the anatomy, the coil sensitivities and the sampling patterns are taken from a neuro scan. The normal data has been replaced with pure random, white noise. Three different ways of thresholding have been applied and the resulting images and corresponding wavelet transforms are shown (right). The color indicates phase, and can be ignored. The three methods are:

- A constant threshold (top right),
- "Laplace", level dependent threshold, the thresholds coming from the normal object,
- "Laplace, energy preserving". This is as "Laplace", but the level has been limited such that, per wavelet scale, only a fixed percentage of the signal is removed.

It can be observed that while the noise for the constant threshold looks natural, the other results show colored noise (see also the corresponding wavelet transforms). Based on these results and many real scans it has been decided to apply a constant threshold only.

Threshold level / wavelet balancing

Let us first list two of the main elements of some examples:

- "The application of the sparsity constraint in CS-SENSE does not require any user interaction". Or more precise: the user does not have to specify the level of thresholding.
- The application of the sparsity constraint does not lead to visible resolution loss.

The first requirement implies that an automatic scheme is required that determines the threshold level, independent from any protocol parameter or other aspect of the scan.

Looking at the relation between the wavelet scales and types, on one hand and k-space on the other hand (section 0), as well as the relation between the Laplace scales and the wavelet scales and types, it is clear that the threshold should be based on the finest wavelet scale+type, that corresponds to measured data. Since the HH type on the finest wavelet scale more or less corresponds to a non-measure (or filtered) part of k-space it should not play a role in the determination of the threshold. Then, given the fact that anisotropic resolution and possible anisotropic aspects of the object itself may cause a significant amplitude difference between LH and HL types, it has been decided to base the threshold on the minimum Laplace scale of these two types, at the finest wavelet scale, of course. In a formula, the Laplace scale used is:

$$b_{th} = \min(b_{s=0}^{LH}, b_{s=0}^{HL}). \qquad (0.23)$$

Now the threshold could follow from this expression. But we also have the restriction that the threshold level should be chosen such that no visible resolution loss occurs as consequence of thresholding. After some educated guessing and supporting experiments, level have been chosen such that no more than 5% or 10% of the energy of the relevant wavelet scale/type is lost. Let us now see how the actual threshold can be derived from this level and the Laplace scale.

Actually a detailed description is beyond the scope of this document. The outline of the derivation is as follows. Given the Laplace scale(s), the inverse problem can be solved using the iterative scheme (0.9) using a threshold value:

$$t_i = \frac{\sigma^2}{bc}, \qquad (0.24)$$

Where the subscript "i" stands for "iterative scheme". Now it would be extremely hard to derive a bound on the threshold from the iterative scheme itself. However, for just a single thresholding step it is possible to derive a bound. The derivation now is based on the fact that actually the CS-SENSE algorithm can exactly be split into a SENSE algorithm, followed by a thresholding step, which uses the covariance matrix of the noise of the output image of the SENSE step. However, for practical results, the covariance matrix has to be approximated. It can be shown that with this approximation, the same problem can be solved in one iteration, with the threshold

$$t_c = \frac{\sigma^2 R}{b}, \qquad (0.25)$$

where the subscript "c" stands for "combined scheme". So these two thresholds are related to each other via

$$t_i = \frac{t_c}{cR}. \qquad (0.26)$$

The next step comes from analyzing the bound on the threshold $t_c$ such that a fraction f of the energy of the signal is lost in the threshold operation (for the combined scheme). It can be derived that this gives a condition:

$$t_c \leq \frac{3bf.}{2} \qquad (0.27)$$

Combining this with relation (0.26) yields the bound on the threshold used in the iterative scheme:

$$t_i \leq \frac{3bf}{2cR}.$$

$$(0.28)$$

This value is used for the iterative scheme.

**Claims**

1. A medical imaging system (100, 300, 500, 600) comprising:

   - a memory (110) for storing machine executable instructions (120);
   - a processor (106) for controlling the medical instrument, wherein execution of the machine executable instructions causes the processor to:

     - receive (200) MRF magnetic resonance data (122) acquired according to an MRF magnetic resonance imaging protocol of a region of interest (310);
     - reconstruct (202) an MRF vector (124) for each voxel of a set of voxels descriptive of the region of interest using the MRF magnetic resonance data according to the MRF magnetic resonance imaging protocol;
     - calculate (204, 714) a preprocessed MRF vector (126) for each of the set of voxels by applying a predetermined preprocessing routine to the MRF vector for each voxel, wherein the predetermined preprocessing routine comprises normalizing the preprocessed MRF vector for each voxel;
     - calculate (206, 716) an outlier map (130) for the set of voxels by assigning an outlier score (800) to the preprocessed MRF vector using a machine learning algorithm.

2. The medical imaging system of claim 1, wherein the medical imaging system further comprises a magnetic resonance imaging system, wherein the memory further comprises MRF pulse sequence commands (340) configured for controlling the magnetic resonance imaging system to acquire the MRF magnetic resonance data from the region of interest, wherein execution of the machine executable instructions cause the processor to control (400, 712) the magnetic resonance imaging system to acquire the MRF magnetic resonance data.

3. The medical imaging system of any one of the preceding claims, wherein execution of the machine executable instructions further causes the processor to receive a segmentation (546, 546') of the set of voxels, wherein the segmentation identifies a voxel type for each of the set of voxels, and wherein the outlier score is at least partially assigned by using the voxel type as an input to the machine learning algorithm.

4. The medical imaging system of claim 3, wherein the voxel type is any one of the following: an anatomical location derived from an annotated anatomical atlas, a tissue type, an organ type, a global voxel, and combinations thereof.

5. The medical imaging system of any one of the preceding claims, wherein execution of the machine executable instructions further causes the processor to reconstruct a magnetic resonance image according to any one of the following:

   - reconstruct the magnetic resonance image (544) from the MRF vector for each voxel using a magnetic resonance fingerprinting dictionary (542); and
   - reconstruct the magnetic resonance image (544') from imaging magnetic resonance data (642), wherein the imaging magnetic resonance data is descriptive of the region of interest.

6. The medical imaging system of claim 5, wherein execution of the machine executable instructions further causes the processor to:

   - identify anomalous voxels by thresholding the outlier map; and
   - render a medical image (550, 550') comprising the magnetic resonance image, and wherein the anomalous voxels are marked in the medical image.

7. The medical imaging system of claim 6, wherein execution of the machine executable instructions further causes the processor to train the machine learning algorithm by:

- receiving (702) training MRF magnetic resonance data acquired according to an MRF magnetic resonance imaging protocol of a training region of interest;
- reconstructing a training MRF vector for each voxel of a set of training voxels descriptive of the training region of interest using the training MRF magnetic resonance data according to the MRF magnetic resonance imaging protocol;
- calculate (704, 706, 708) a training preprocessed MRF vector for each of the training set of voxels by applying the predetermined preprocessing routine to the MRF vector for each voxel; and
- train (710) the machine learning algorithm using the training preprocessed MRF vector.

8. The medical imaging system of any one of the preceding claims, wherein the predetermined preprocessing routine comprises reducing the dimensionality of the MRF vector for each voxel.

9. The medical imaging system of claim 8, wherein reducing the dimensionality of the MRF vector for each voxel comprises any one of the following:

- applying a Fourier transform to the MRF vector and truncating the Fourier transformed MRF vector above a predetermined frequency value;
- condensing the MRF vector using a principal components analysis algorithm; and
- calculating multiple relaxation times using an MRF dictionary.

10. The medical imaging system of any one of the preceding claims, wherein the predetermined preprocessing routine comprises any one of the following:

- applying a mask to remove chosen voxels from the set of voxels;
- deleting the chosen voxels from the set of voxels if the MRF vector is below a predetermined amplitude or a predetermined measure; and
- combinations thereof.

11. The medical imaging system of any one of the preceding claims, wherein the trained machine learning algorithm is an outlier detection algorithm.

12. The medical imaging system of any one of the preceding claims, wherein the machine learning algorithm is any one of the following: an Isolation Forest algorithm, a k Nearest Neighbors algorithm, and a one-class support vector machine algorithm.

13. A computer program product comprising machine executable instructions (120) for execution by a processor (106), wherein execution of the machine executable instructions causes the processor to:

- receive (200) MRF magnetic resonance data (122) acquired according to an MRF magnetic resonance imaging protocol of a region of interest (310);
- reconstruct (202) an MRF vector (124) for each voxel of a set of voxels descriptive of the region of interest using the MRF magnetic resonance data according to the MRF magnetic resonance imaging protocol;
- calculate (204, 714) a preprocessed MRF vector (126) for each of the set of voxels by applying a predetermined preprocessing routine to the MRF vector for each voxel, wherein the predetermined preprocessing routine comprises normalizing the preprocessed MRF vector for each voxel;
- calculate (206, 716) an outlier map (130) for the set of voxels by assigning an outlier score (800) to the preprocessed MRF vector using a machine learning algorithm.

14. A method of operating a medical imaging system (100, 300, 500, 600), wherein the method comprises:

- receiving (200) MRF magnetic resonance data (122) acquired according to an MRF magnetic resonance imaging protocol of a region of interest (310);
- reconstructing (202) an MRF vector (124) for each voxel of a set of voxels descriptive of the region of interest using the MRF magnetic resonance data according to the MRF magnetic resonance imaging protocol;
- calculating (204, 714) a preprocessed MRF vector (126) for each of the set of voxels by applying a predetermined preprocessing routine to the MRF vector for each voxel, wherein the predetermined preprocessing routine comprises normalizing the preprocessed MRF vector for each voxel;
- calculating (206, 716) an outlier map (130) for the set of voxels by assigning an outlier score (800) to the preprocessed MRF vector using a machine learning algorithm.

Fig. 1

Fig. 2

Fig. 3

Fig. 4

Fig. 5

Fig. 6

Fig. 7

Fig. 8

Fig. 9

Fig. 10

Fig. 11

Fig. 12

Fig. 13

Europäisches
Patentamt

European
Patent Office

Office européen
des brevets

# EUROPEAN SEARCH REPORT

Application Number

EP 18 16 3288

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2017/007696 A1 (TESLA HEALTH INC [US]; STEVENS ANDREW G [US]) 12 January 2017 (2017-01-12) * paragraph [0012] * * paragraph [0037] - paragraph [0039] * * paragraph [0048] * * paragraph [0050] * * paragraph [0062] - paragraph [0063] * * paragraph [0074] * * paragraph [0116] * * paragraph [0127] * * paragraph [0129] * * paragraph [0142] - paragraph [0143] * * paragraph [0156] - paragraph [0157] * * paragraph [0164] * * paragraph [0184] * * paragraph [0216] - paragraph [0217] * * paragraph [0222] * * paragraph [0240] * * paragraph [0243] - paragraph [0244] * * figure 1 * ----- | 1-14 | INV. G06K9/66 G01R33/56 G06K9/68 ADD. A61B5/055 A61B6/00 |
| A | WO 2015/160400 A2 (GEN HOSPITAL CORP [US]) 22 October 2015 (2015-10-22) * paragraph [0003] - paragraph [0004] * * paragraph [0006] - paragraph [0007] * * paragraph [0012] * * paragraph [0019] - paragraph [0020] * * paragraph [0024] - paragraph [0025] * * figure 1 * ----- | 1-6,10, 11,13,14 | TECHNICAL FIELDS SEARCHED (IPC) G06K A61B G01R |
| A | WO 2017/072034 A1 (KONINKLIJKE PHILIPS NV [NL]) 4 May 2017 (2017-05-04) * page 5, line 26 - page 6, line 16 * * page 8, line 3 - line 16 * * page 15, line 4 - line 22 * ----- -/-- | 1-5,13, 14 | |

The present search report has been drawn up for all claims

| Place of search | Date of completion of the search | Examiner |
|---|---|---|
| The Hague | 29 August 2018 | Moreno, Marta |

EPO FORM 1503 03.82 (P04C01)

page 1 of 2

Europäisches
Patentamt

European
Patent Office

Office européen
des brevets

## EUROPEAN SEARCH REPORT

Application Number

EP 18 16 3288

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2017/103287 A1 (HAN XIAO [US]) 13 April 2017 (2017-04-13) <br> * paragraph [0034] * <br> * paragraph [0038] - paragraph [0041] * <br> * paragraph [0058] - paragraph [0059] * <br> * paragraph [0063] * <br> ----- | 1,8,9, 13,14 | |
| A | US 2016/139227 A1 (GRODZKI DAVID [DE] ET AL) 19 May 2016 (2016-05-19) <br> * paragraph [0002] * <br> * paragraph [0005] * <br> * paragraph [0009] - paragraph [0012] * <br> * paragraph [0021] - paragraph [0024] * <br> * paragraph [0033] - paragraph [0037] * <br> * paragraph [0040] * <br> * figure 2 * <br> ----- | 1,2,5, 10,13,14 | |

TECHNICAL FIELDS
SEARCHED     (IPC)

The present search report has been drawn up for all claims

| Place of search | Date of completion of the search | Examiner |
|---|---|---|
| The Hague | 29 August 2018 | Moreno, Marta |

CATEGORY OF CITED DOCUMENTS

X : particularly relevant if taken alone
Y : particularly relevant if combined with another
  document of the same category
A : technological background
O : non-written disclosure
P : intermediate document

T : theory or principle underlying the invention
E : earlier patent document, but published on, or
  after the filing date
D : document cited in the application
L : document cited for other reasons

& : member of the same patent family, corresponding
  document

EPO FORM 1503 03.82 (P04C01)

page 2 of 2

## ANNEX TO THE EUROPEAN SEARCH REPORT
## ON EUROPEAN PATENT APPLICATION NO.

EP 18 16 3288

This annex lists the patent family members relating to the patent documents cited in the above-mentioned European search report.
The members are as contained in the European Patent Office EDP file on
The European Patent Office is in no way liable for these particulars which are merely given for the purpose of information.

29-08-2018

| Patent document cited in search report | | Publication date | Patent family member(s) | | Publication date |
|---|---|---|---|---|---|
| WO 2017007696 | A1 | 12-01-2017 | US 2017007148 A1 | | 12-01-2017 |
| | | | WO 2017007696 A1 | | 12-01-2017 |
| WO 2015160400 | A2 | 22-10-2015 | CN 106537168 A | | 22-03-2017 |
| | | | EP 3105605 A2 | | 21-12-2016 |
| | | | KR 20160119807 A | | 14-10-2016 |
| | | | US 2016349341 A1 | | 01-12-2016 |
| | | | WO 2015160400 A2 | | 22-10-2015 |
| WO 2017072034 | A1 | 04-05-2017 | CN 108351395 A | | 31-07-2018 |
| | | | DE 112016004907 T5 | | 05-07-2018 |
| | | | WO 2017072034 A1 | | 04-05-2017 |
| US 2017103287 | A1 | 13-04-2017 | AU 2016339009 A1 | | 26-04-2018 |
| | | | EP 3362146 A1 | | 22-08-2018 |
| | | | US 2017103287 A1 | | 13-04-2017 |
| | | | WO 2017066317 A1 | | 20-04-2017 |
| US 2016139227 | A1 | 19-05-2016 | DE 102014223388 A1 | | 19-05-2016 |
| | | | US 2016139227 A1 | | 19-05-2016 |

EPO FORM P0459

For more details about this annex : see Official Journal of the European Patent Office, No. 12/82

**EP 3 543 911 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **MA et al.** Magnetic Resonance Fingerprinting. *Nature,* 1971, vol. 495, 187-193